(19) Europäisches Patentamt
European Patent Office
Office européen des brevets

(11) **EP 4 160 488 A1**

(12) **EUROPEAN PATENT APPLICATION**

(43) Date of publication:
**05.04.2023 Bulletin 2023/14**

(21) Application number: **22197895.0**

(22) Date of filing: **27.09.2022**

(51) International Patent Classification (IPC):
**G06N 3/098** (2023.01)     **G06N 3/084** (2023.01)
**G06N 3/0464** (2023.01)    **G06N 3/09** (2023.01)
**G16H 30/40** (2018.01)

(52) Cooperative Patent Classification (CPC):
**G06N 3/0464; G06N 3/084; G06N 3/09;**
**G06N 3/098;** G16H 30/40

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **28.09.2021 US 202117449165**

(71) Applicant: **Siemens Healthcare GmbH**
**91052 Erlangen (DE)**

(72) Inventors:
• **YOO, Youngjin**
**Princeton, NJ 08540 (US)**

• **GIBSON, Eli**
**Plainsboro, NJ 08536 (US)**
• **PATEL, Pragneshkumar**
**East Windsor, NJ 08512 (US)**
• **PALADINI, Gianluca**
**Skillman, NJ 08558 (US)**
• **ULLASKRISHNAN, Poikavila**
**Lebanon, NH 03766 (US)**
• **COMANICIU, Dorin**
**Princeton, NJ 08540 (US)**

(74) Representative: **Horn Kleimann Waitzhofer**
**Patentanwälte PartG mbB**
**Ganghoferstraße 29a**
**80339 München (DE)**

(54) **ADAPTIVE AGGREGATION FOR FEDERATED LEARNING**

(57)     Systems and Methods for adaptive aggregation in a federated learning model. An aggregation server (121) sends global model weights to all chosen collaborators (131) for initialization. Each collaborator (131) updates the model weights for certain epochs and then sends the updated model weights back to the aggregation server (121). The aggregation server (121) adaptively aggregates the updated model weights using at least a computed model divergence value and sends the aggregated model weight to collaborators (131).

A110 — Receive model parameters

A120 — Calculate divergence value

A130 — Aggregate model parameters

A140 — Transmit aggregated model parameters

FIG. 2

## Description

FIELD

The present embodiments relate to federated learning.

BACKGROUND

In the last few years deep learning has been elevated from an area of medical research into an area of medical products because of improvements in computing hardware and proliferation of healthcare data. Massive volumes of data are collected every day by large number of entities such as research centers, hospitals, or other medical entities. Analysis of the data could improve learning models and user experiences. The complex problem of training these models could be solved by distributed computing by taking advantage of the resource storage, computing power, cycles, content, and bandwidth of participating devices available at the edges of a network. In such a distributed machine learning scenario, the dataset is transmitted to or stored among multiple edge devices. The devices solve a distributed optimization problem to collectively learn the underlying model. For distributed computing, similar (or identical) datasets may be allocated to multiple devices that are then able to solve a problem in parallel. However, access to large, diverse healthcare datasets remains a challenge due to regulatory concerns over sharing protected healthcare information.

Privacy and connectivity concerns may prohibit data from being shared between entities preventing largescale distributed methods. Hospitals, for example, may prefer not to or may not be allowed to share medical data with other entities or unknown users. Federated learning is a distributed computing approach that enables entities to collaborate on machine learning projects without sharing sensitive data such as patient records, financial data, or classified secrets. The basic premise behind federated learning is that the model moves to meet the data rather than the data moving to meet the model. Therefore, the minimum data movement needed across the federation is solely the model parameters and their updates. Challenges still exist though in managing the flow of data, the training of models, and privacy issues.

SUMMARY

Systems, methods, and computer readable media are provided for adaptive aggregation of model parameters in a federated learning environment.

In a first aspect, a method is provided for aggregating parameters from a plurality of collaborator devices in a federated learning system that trains a model over multiple rounds of training, for each round of training the method comprising: receiving model parameters from two or more of the plurality of collaborator devices; calculating for each of the two or more collaborator devices a model divergence value that approximates how much an updated collaborator model for a respective collaborator device of the two or more collaborator devices deviates from a prior aggregated model; aggregating model parameters for the model from the received model parameters based at least on the respective model divergence value for each collaborator; and transmit the aggregated model parameters to the plurality of collaborator devices; wherein the aggregated model parameters are used by the plurality of collaborator devices for a subsequent round of training.

In a second aspect, a system is provided for federated learning. The system includes a plurality of collaborators and an aggregation server. Each collaborator of the plurality of collaborators is configured to train a local machine learned model using locally acquired training data, update local model weights for the local machine learned model, and send the updated local model weights to an aggregation server. The aggregation server is configured to receive the updated model weights from the plurality of collaborators, calculate a model divergence value for each collaborator from respective updated model weights and a prior model, calculate aggregated model weights based at least in part on the model divergence values, and transmit the aggregated model weights to the plurality of collaborators to update the local machine learned model.

In a third aspect, an aggregation server for federated learning of a model. The aggregation server includes a transceiver, a memory, and a processor. The transceiver is configured to communicate with a plurality of collaborator devices. The memory is configured to store model parameters for the model. The processor is configured to receive model parameters from the plurality of collaborator devices, calculate for each collaborator device of the plurality of collaborator devices a model divergence value, aggregate the model parameters at least in part based on the model divergence values, and transmit the aggregated model parameters to the plurality of collaborator devices.

It is understood that the aggregation server of the second and/or the processor of the third aspect, in embodiments, is implemented to carry out the method steps according to the first method as depicted above or below and/or as indicated in the individual claims. Model weights may be considered model parameters.

Any one or more of the aspects described above may be used alone or in combination. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.

Figure 1 depicts an example federated learning system.

Figure 2 depicts an example method for adaptive aggregation of model parameters according to an embodiment.

Figure 3 depicts an example workflow for adaptive aggregation of model parameters according to an embodiment.

Figure 4 depicts an example aggregation server according to an embodiment.

DETAILED DESCRIPTION

Embodiments provide systems and methods for adaptive aggregation in a federated learning model. An aggregation server sends global model weights to all chosen collaborators for initialization. Each collaborator updates the model weights for certain epochs and then sends the updated model weights back to the aggregation server. The aggregation server adaptively aggregates the updated model weights using at least a computed model divergence value and send the aggregated model weight to collaborators. By estimating model divergence for each collaborator at each round using a preserved model divergence testing dataset, embodiments provide robust federated learning for non-homogenous datasets between participating collaboration sites.

Federated learning (FL) is a distributed approach for training a model that includes multiple distributed devices and at least one aggregation server or device. Each of the devices download a current model and computes an updated model at the device itself (ala edge computing) using local data. The locally trained models are then sent from the devices back to the central server where the models or parameters are aggregated. A single consolidated and improved global model is sent back to the devices from the server. Federated learning allows for machine learning algorithms to gain experience from a broad range of data sets located at different locations. The approach enables multiple organizations to collaborate on the development of models, but without needing to directly share secure data with each other. Over the course of several training iterations, the shared models get exposed to a significantly wider range of data than what any single organization possesses in-house. In other words, federated learning decentralizes machine learning by removing the need to pool data into a single location. Instead, the model is trained in multiple iterations at different locations.

In an embodiment, the collaborators or remote locations include hospitals and medical centers. With federated learning, these sites can remain in full control and possession of their patient data with complete traceability of data access, limiting the risk of misuse by third parties. Existing medical data is typically not fully used by machine learning because the data resides in data silos or walled gardens and privacy concerns restrict access. Centralizing or releasing data, however, poses not only regulatory, ethical, and legal challenges, related to privacy and data protection, but also technical ones. Anonymizing, controlling access and safely transferring healthcare data is a non-trivial, and sometimes impossible task. Anonymized data from the electronic health record can appear innocuous and compliant with regulations, but just a few data elements may allow for patient reidentification. The same applies to genomic data and medical images making them as unique as a fingerprint. Therefore, unless the anonymization process destroys the fidelity of the data, likely rendering it useless, patient reidentification or information leakage cannot be ruled out. Hospitals and medical centers may thus make great use of federated learning.

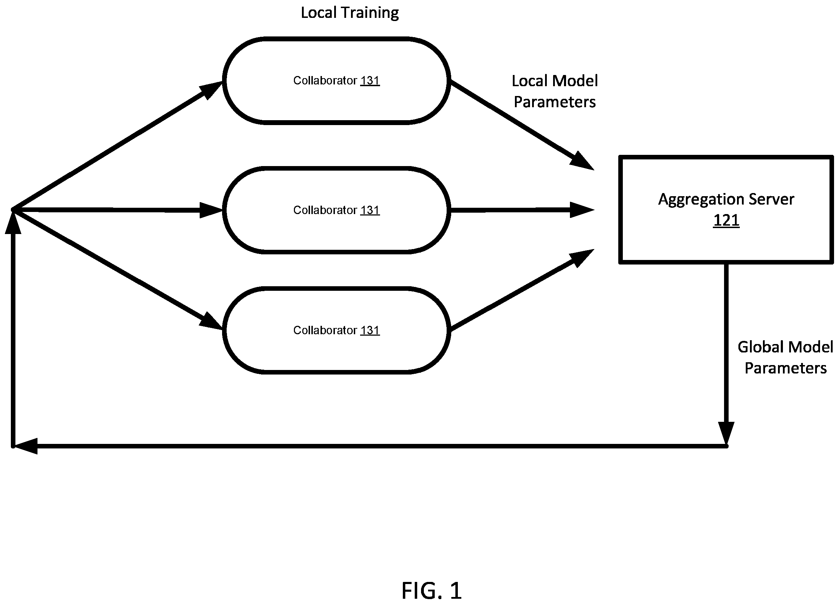

Figure 1 depicts an example of a federated learning system. As depicted, the federated learning system includes three collaborator devices / remote devices herein also referred to as collaborators 131 and a central / aggregation server 121. In a typical federated learning system, there may be tens, hundreds, thousands, or more clients / collaborators 131 depending on the application. Each collaborator 131 is configured to acquire local data for which to locally train a model over multiple rounds. To start the training, the aggregation server 121 sends global model parameters (for example model weights) to all chosen collaborators 131 for initialization. Each collaborator 131 trains a local model (initialized with the global model parameters) with locally acquired data and updates the model parameters for certain epochs. The collaborators 131 then send updated model parameters back to the aggregation server 121. The aggregation server 121 aggregates the updated model parameters and then send the global model parameters to collaborators 131 for another round of training. The result is a system that allows the training data to remain securely onsite which increases both security and privacy. For certain models such as medical applications, data is now available to be used when previously privacy concerns and regulations may have prohibited its use. There are, however, potential drawbacks and complications that arise from the distributed nature of federated learning. One issue is with the aggregation step at the aggregation server 121, e.g., how best to aggregate the model updates from the different collaborators 131.

The aggregation of the parameters typically uses an averaging mechanism. For example, one popular approach is Federated Averaging (FedAvg) where the model weights of the different local models are averaged by the aggregation server 121 to provide new model weights and, thus, a new aggregated model. In an example, at each iteration, FedAvg first locally performs epochs of stochastic gradient descent (SGD) on the devices. The devices then communicate their model updates to the aggregation server 121, where they are averaged. FedAvg and other parameter averaging mechanisms work well when each device possesses similar amounts and types of data for which to train the model. However, this is typically not the case in the real world. The data on each device may be non-independently and identically distributed data (non-IID). Identically distributed means that there are no overall trends-the distribution doesn't fluctuate and all items in the samples are taken from the same probability distribution. Non-IID data is generally heterogeneous data that may vary in quality, content, and quantity. In an example, one site or collaborator 131 may acquire poor quality data while another collaborator 131 may acquire high quality data. If weights from the two collaborators 131 where just averaged, the result would be subpar. Similarly, if a first site has large amounts of data and another site has small amounts of data, the weights from the first site may be more relevant in generating a quality model.

A deterioration in accuracy of FL is inevitable on non-IID or heterogeneous data. The performance degradation may mainly be attributed to weight divergence of the local models resulting from non-IID. That is, local models having the same initial parameters will converge to different models because of the heterogeneity in local data distributions. During the process of federated learning the divergence between the shared global model acquired by averaging the uploaded local models and the ideal model (the model obtained when the data on the local devices is IID) continues to increase, slowing down the convergence and worsening the learning performance.

Embodiments described herein provide for distributed processing of data while maintaining privacy and transmission concerns. The training occurs in a decentralized manner with multiple collaborators 131 with only the local data available to each collaborator 131. The multiple collaborators 131 do not share data. The aggregation of model parameters occurs on an aggregation server 121. Aggregation is performed by weighted average, in which the aggregation weight is adaptively computed. Embodiments utilize a model divergence value that approximates how much an updated collaborator model deviates from a previous aggregated model and adjusts aggregation weights based on the approximated divergence. The model divergence may be caused by non-IID samples, heterogeneity, and class imbalance in a collaborator's data, that may disturb the performance and stability of aggregation at each communication round. The model divergence is estimated by measuring aggregation testing metrics for each collaborator 131 at each communication round. Embodiments utilize a preserved test dataset and a collaborator model from the current round to compute the global testing performance for each collaborator 131. The model divergence can be adjusted by combining with L2-norm or L1-norm of difference between an updated collaborator model and the previous aggregated model. Aggregation weights are computed for each collaborator 131 based on the estimated divergence. Additionally, aggregation weights may be further adjusted by class imbalance ratio (for example a ratio between positive and negative samples) and number of data samples of each collaborator 131. The updated aggregated model is then sent back to each collaborator 131 for the next round.

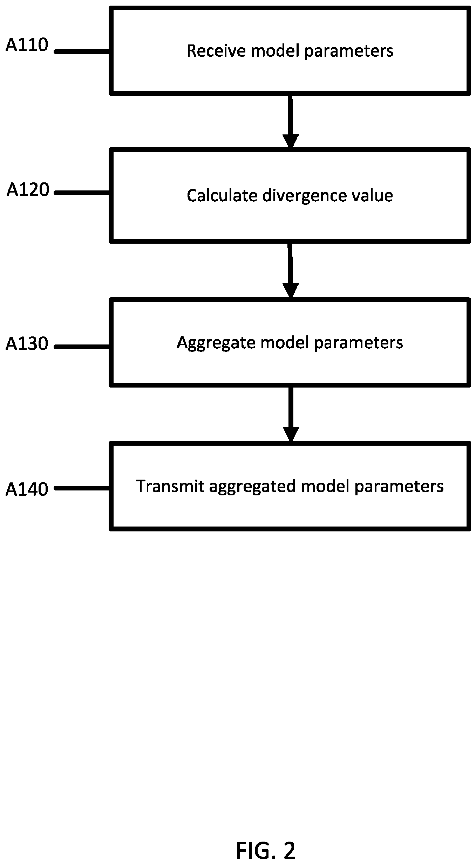

Figure 2 depicts a method for aggregating model parameters from a plurality of collaborators 131 in a federated learning system that trains a model over multiple rounds of training. The workflow describes one potential round of multiple rounds of training that are to be performed. There may be tens, hundreds, thousands, or more rounds performed until the model is trained. For each round, the collaborators 131 or remote devices train a local model with local data. The parameters are then sent to an aggregation server 121 / aggregator that is configured to aggregate the parameters from multiple collaborators 131 into a single central model. The parameters for the single central model are transmitted to the collaborators 131 for a subsequent round of training. As presented in the following sections, the acts may be performed using any combination of the components indicated in Figure 1, 3, or 4. The following acts may be performed by the collaborators 131, an aggregation server 121, a cloud-based server, or a combination thereof. Additional, different, or fewer acts may be provided. The acts are performed in the order shown or other orders. The acts may also be repeated. Certain acts may be skipped.

At Act A110, the aggregation server 121 receives model parameters from two or more of the plurality of collaborators 131. The collaborators 131 may be remotely located from the aggregation server 121. In an embodiment, the collaborators 131 are hospital sites. The collaborators 131 are configured to train a model using locally acquired data that for privacy or security reasons does not leave the collaborator 131. Each collaborator 131 acquires data, trains a model for one or more epochs, and then transmits model parameters to the aggregation server 121 over a network. The centralized server / aggregation server 121 may include one or more machines or servers. A hierarchy of aggregation servers may be used to receive the model parameters which may be further aggregated by an additional server. The aggregation servers 121 may be configured to operate in the cloud or on multiple different machines. In an embodiment, the aggregation server 121 and collaborators 131 are remotely located. Alternatively, the aggregation server 121 and collaborators 131 may be co-located. The aggregation server 121 and collaborators 131 communicate using the network that may include wired networks, wireless networks, or combinations thereof. The wireless network may be a cellular telephone network, LTE (Long-Term Evolution), 4G LTE, a wireless local area network, such as an 802.11, 802.16, 802.20, WiMax (Worldwide Interoperability for Microwave Access) network, DSRC (otherwise known as WAVE, ITS-G5, or 802.11p and future generations thereof), a 5G wireless network, or wireless short-range network. Further, the network 127 may be a public network, such as the Internet, a private network, such as an intranet, or combinations thereof, and may utilize a variety of networking protocols now available or later developed including, but not limited to transmission control protocol/internet protocol (TCP/IP) based networking protocols.

The model may be any model that is trained using a machine learning process. Examples for medical applications include finding clinically similar patients, predicting hospitalizations due to cardiac events, mortality and ICU stay time. Models may also include applications in the field of medical imaging such as for whole-brain segmentation as well as brain tumor segmentation, for example. In an embodiment, the model may be used to identify disease-related biomarkers in the context of COVID-19.

The model parameters may be represented by parameter vectors. A parameter vector may be a collection (e.g., set) of parameters from the model or a representation of the set of parameters. The parameter vector may be a randomly chosen components of a parameter vector. Models may include thousands or millions of parameters. Compressing the set of parameters into a parameter vector may be more efficient for bandwidth and timing than transmitting and recalculating each parameter of the set of parameters. A parameter vector may also be further compressed. In an embodiment, an incoming parameter vector I may also be compressed into a sparse subspace vector.

In an embodiment, the training data on each of the collaborators 131 is not independently and identically distributed (non-I.I.D.). The distribution of data for two different collaborators 131 is different and unbalanced (for example, the collaborators 131 have different orders of magnitudes of acquired data). In an example, for image data, one device may have several gigabytes of medical imaging data that relates to images taken for multiple procedures for multiple patients while another has only a single set of image data. Both sets of data may be useful to train a segmentation model though the collaborator 131 with more data may provide more useful parameters. The quality of data may also differ between devices. Certain devices may include higher quality sensors or may include more storage for data allowing higher quality data to be captured.

The collaborators 131 are configured to train or configure a local model using the training data. In an embodiment, the training data is labeled. Labeled data is used for supervised learning. The model is trained by imputing known inputs and known outputs. Weights or parameters are adjusted until the model accurately matching the known inputs and output. In an example, to train a machine learned model to identify certain artifacts using acquired image data, images of the artifacts - with a variety of configurations - are required as input variables. The labels, e.g., the correct designations, for such data may be assigned manually or automatically. The correct set of input variables and the correct classifications constitute the training data set. Labels may be provided by, for example, requesting additional input from a user (requesting a manual annotation), derived from additional data (parsing textual descriptions), or by incorporating additional data from other sources.

Other methods for labeling data may be used, for example, a cloud-based service may give accurate, albeit incomplete, labels that be downloaded from the cloud to the device. In an embodiment, the training data is labeled, and the model is taught using a supervised learning process. A supervised learning process may be used to predict numerical values (regression) and for classification purposes (predicting the appropriate class). A supervised learning processing may include processing images, audio files, videos, numerical data, and text among other types of data. Classification examples include segmentation, object recognition, face recognition, credit risk assessment, voice recognition, and customer churn, among others. Regression examples include determining continuous numerical values on the basis of multiple (sometimes hundreds or thousands) input variables.

The model may be any model that is trained using a machine learned process. The model may include machine learned processes such as support vector machine (SVM), boosted and bagged decision trees, k-nearest neighbor, Naive Bayes, discriminant analysis, logistic regression, and neural networks. In an example, a two-stage convolutional neural network is used that includes max pooling layers. The two-stage convolutional neural network (CNN) uses rectified linear units for the non-linearity and a fully connected layer at the end for image classification. In an embodiment, the model may be trained using an adversarial training process, e.g., the model may include a generative adversarial network (GAN). For an adversarial training approach, a generative network and a discriminative network are provided for training by the devices. The generative network is trained to identify the features of data in one domain A and transform the data from domain A into data that is indistinguishable from data in domain B. In the training process, the discriminative network plays the role of a judge to score how likely the transformed data from domain A is similar to the data of domain B, e.g., if the data is a forgery or real data from domain B.

In an embodiment, the model is trained using a gradient descent technique or a stochastic gradient descent technique. Both techniques attempt to minimize an error function defined for the model. For training (minimizing the error function), a collaborator 131 first connects to the parameter server. The collaborator 131 may start with randomly initialized model parameters or may request initial model parameters from the parameter server. The starting parameters may also be derived from another, pretrained model rather than being randomly initialized. The initial parameters may be assigned to all subsequent collaborators 131. Alternatively, updated central parameters may be assigned if the training process has already begun. In an example, collaborators 131 may initially communicate with the parameter server at different times. A first collaborator 131 may communicate with the aggregation server 121 and be assigned randomly initialized model parameters. Similarly, a second collaborator 131 may communicate shortly thereafter with the aggregation server 121 and be assigned randomly initialized model parameters. At some point, the collaborators 131 begin transmitting model parameters back to the aggregation server 121. As detailed below, the aggregation server 121 updates the central model parameters and transmits the updated model parameters back to the collaborators 131. Any collaborator 131 that first communicates with the parameter server after this time may be assigned the central parameters and not the randomly initialized model parameters. In this way, new collaborators 131 may be added to the system at any point during the training process without disrupting the training process. Handing out the latest parameters to newly joined collaborators 131 may result in faster learning at early stages.

The gradient descent technique attempts to minimize an error function for the model. Each collaborator 131 trains a local model using local training data. Training the model involves adjusting internal weights or parameters of the local model until the local model is able to accurately predict the correct outcome given a newly input data point. The result of the training process is a model that includes one or more local parameters that minimize the errors of the function given the local training data. The one or more local parameters may be represented as a parameter vector. As the local training data is limited the trained model may not be very accurate when predicting the result of an unidentified input data point. The trained model, however, may be trained to be more accurate given starting parameters that cover a wider swath of data. Better starting parameters may be acquired from the aggregation server 121.

Referring back to Figure 2, at act A120, the aggregation server 121 calculates for each of the two or more collaborators 131 a model divergence value that approximates how much an updated collaborator model for a respective collaborator 131 of the two or more collaborators 131 deviates from a prior aggregated model. The model divergence is estimated by measuring aggregation validation metrics for each collaborator 131 at each communication round. A separately preserved validation dataset is compared with a collaborator model from the current round to compute the validation metric for each collaborator 131. The prior aggregated model may be the model or aggregated parameters from the previous round. The divergence may be calculated, for example, using a L1 norm or L2 norm.

The L1 norm is calculated as the sum of the absolute values of a vector for the respective model parameters. The L2 norm is calculated as the square root of the sum of the squared vector values, for example using the equation: $\left\| \mathbf{w}_{aggregated} - \mathbf{w}_c \right\|_2^2$ , where c indicates collaborators 131). By estimating model divergence for each collaborator 131 at each round using a preserved model divergence testing dataset, embodiments perform adaptive aggregation that can be robust to non-IID datasets between participating collaboration sites. In an example, if one collaborator's model parameters diverge a significant amount from the previous model, the model parameters may be weighed lower than a different collaborator 131 that is more similar to the previous model. In this way, outliers that are generated as a result of non-IID data may be diminished and not allowed to take over the direction of the training.

At act A130, the aggregation server 121 aggregates model parameters for the model from the received model parameters based at least on the respective model divergence value for each collaborator 131. The aggregation of the model parameters includes a weighting of the locally trained model parameters to the centrally stored model parameters that may be based on the number of data points, the staleness of the updates, and the data distribution (e.g., unbalanced non-I.I.D.). An adaptive aggregation is used to account for the number of data points, the staleness of the parameter updates, and the data distribution. In an embodiment, a model divergence value that approximates how much an updated collaborator model deviates from the previous aggregated model is used to adjust aggregation weights based on the approximated divergence. Additional mechanisms may be used to adaptively adjust the aggregation weights. In an embodiment, a class imbalance ratio (i.e., ratio between positive and negative samples) may be used to update the aggregation weights. Applying a class imbalance ratio to local collaborator 131 training may be important for managing model divergence particularly in FL of disease classification and detection networks to deal with severe class imbalance in collaborating institutions which can cause instable FL progress. In an embodiment, the aggregation weights may also depend on a number of data points from each collaborator 131. Collaborators 131 with more data may be accorded a higher weight when calculating the aggregated parameters for the central model.

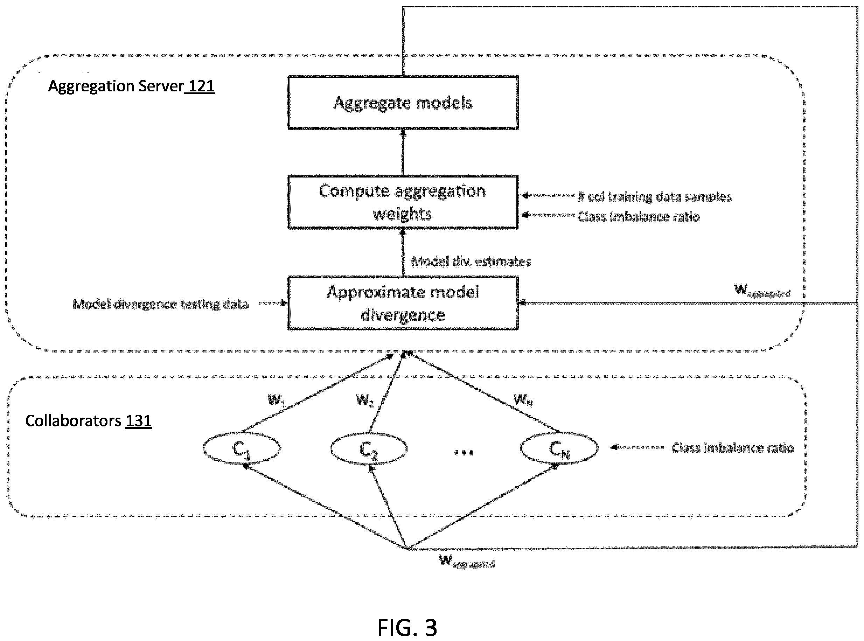

Figure 3 depicts an example of the computation of the aggregation weights. In Figure 3, the collaborators 131 generate parameters ($W_1$, $W_2$, $W_N$) that are received by the Aggregation server 121. The Aggregation server 121 computes an approximate model divergence using aggregated parameters from a previous round ($W_{aggregated}$) and model divergence testing data. The model divergence testing data may include thresholds or values that define an acceptable amount of divergence. The aggregation server 121 computes aggregated weights for each of the parameters ($W_1$, $W_2$, $W_N$) using the model divergence estimates and optionally the number of training data samples and class imbalance ratio. The weights are used to generate an aggregated model that includes the aggregated weights. The aggregated weights are transmitted back to the collaborators 131 and used in a subsequent round when calculating the approximate model divergence.

At act A140, the aggregation server 121 transmits the aggregated model parameters to the plurality of collaborators 131. The aggregated model parameters are used by the plurality of collaborators 131 for a subsequent round of training. The subsequent round is similar to the described round of A110-A140. The difference for each iteration is a different starting point for one or more of the parameters in the model. The central parameter vector that is received may be different than the local parameter vector provided in A110. The process repeats for a number of iterations until the parameters converge or a predetermined number of iterations are reached. This process may be repeated hundreds or thousands of times. In an example, several hundred (e.g., 100 to 500) or thousand (e.g., 3,000 to 5,000) iterations may be performed. Depending on the complexity of the model and the type and quantity of devices and data, more or fewer iterations may be performed. If new data is added to the training data, the device may retrain the model and request a new central parameter (and the process may be fully or partially repeated). The result of the training process is a model that may be able to, for example, accurately predict a classification given an unlabeled input. The model is used on new data to generate, for example, a prediction or classification. In an example, for an image classification model, the collaborator 131 identifies an object or feature in newly acquired (unseen) imaging data using the trained machine learned model.

In an embodiment, the adaptive aggregation method may be applied to training a model for use in medical applications. An example model and method for training is described below. In medical applications, deep learning has the potential to create significant tools for the screening and diagnosis of medical issues, for example recently COVID-19. Nevertheless, access to large, diverse healthcare datasets remains a challenge due to regulatory concerns over sharing protected healthcare information. Although FL has demonstrated promising premises for developing AI-enabled analytic tools in medical imaging without sharing patient information, technical problems and pitfalls in practical settings are still poorly understood which hinders its clinical adoption.

In an embodiment, adaptive aggregation is used to overcome previous issues with the application of FL to COVID-19 diagnosis. One trained model uses a chest computed tomography (CT) scan that is more sensitive for COVID-19 diagnosis and is currently widely applied for early screening of the disease. A segmentation network is used that can automatically quantify abnormal computed tomography (CT) patterns commonly present in COVID-19 patients. A second trained model uses a classification network that can automatically detect COVID-19 pathology and differentiate from other pneumonias, interstitial lung diseases (ILD) and normal subjects in chest CTs.

In an embodiment, the segmentation network includes a U-Net resembling architecture with 3D convolution blocks containing either 1 3 3 or 3 3 3 CNN kernels to deal with anisotropic resolutions. The 3D input tensor is fed into a 3D 1 3 3 convolutional layer followed by batch normalization and leaky ReLU. The feature maps were then propagated to 5 DenseNet blocks. For the first two DenseNet blocks, the features are downsampled by a 1 2 2 convolution with a stride of 1 2 2. The anisotropic downsampling kernels are configured to preserve the inter-slice resolution of input image volumes. The last three DenseNet blocks include isotropic downsampling kernels with the stride of 2 2 2. The input to each decoder block is obtained by concatenating the encoder output features with the same resolution and the feature maps upsampled from the previous decoder. The upsampling kernels are built with transpose convolutional kernels with the sizes and strides same to the corresponding DenseNet blocks. The final network output is derived by projecting the feature maps to 2 output channels and activated by softmax operation.

In an embodiment, the classification network is configured for COVID-19 pathology differentiation on chest CT data, that extracted both 2D axial features and 3D global features. The network includes a ResNet50 as the backbone axial feature extractor, that takes a series of CT in-plane slices as input and generated feature maps for the corresponding slices. The extracted features from all slices are then combined by a max-pooling operation. The global feature is fed to a fully connected layer that produces a COVID-19 prediction score per case by softmax operation.

For each model, during the federated learning, a aggregation server 121 uses adaptive aggregation to deal with the non-IID data from each hospital site. A model divergence value that approximates how much an updated collaborator model deviates from the previous aggregated model is used to adjust aggregation weights based on the approximated divergence. The model divergence was estimated by measuring aggregation validation metrics for each collaborator 131 at each communication round. A separately preserved validation dataset is used with a collaborator model from the current round to compute the validation metric for each collaborator 131. In addition, class imbalance ratio (i.e., ratio between positive and negative samples) are also used to update the aggregation weights.

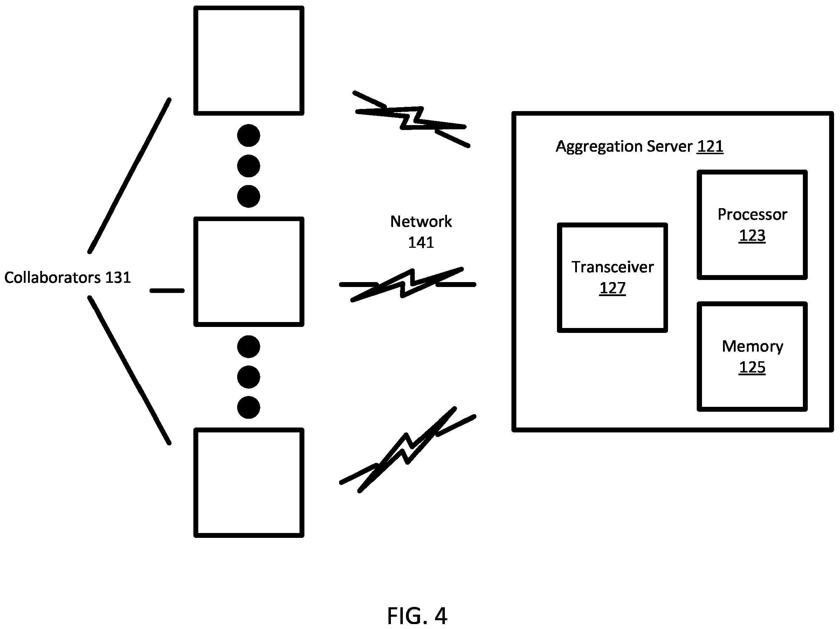

Figure 4 depicts an example of an aggregation server 121. The aggregation server 121 includes at least a memory 125, a processor 123, and a transceiver 127. The aggregation server 121 may communicate with one or more collaborators 131 or sites using the transceiver 127 to acquire model parameters. The one or more collaborators 131 may include hospital sites or centers otherwise equipped to acquire or store medical data for patients. For example, the one or more collaborators 131 may include medical imaging devices and/or PACS systems configured to acquire or store medical imaging data for use in training a model and generating model parameters. The aggregation server 121 is configured to adaptively weight the acquired model parameters using one or more metrics and aggregate the parameters using the adaptive weights. The aggregation server 121 is configured to transmit the aggregated parameters (global parameters) to the one or more collaborators 131 using the transceiver 127.

The memory 125 may be a non-transitory computer readable storage medium storing data representing instructions executable by the processor 123 for time-varying readmission risk prediction. The instructions for implementing the processes, methods and/or techniques discussed herein are provided on non-transitory computer-readable storage media or memories, such as a cache, buffer, RAM, removable media, hard drive, or other computer readable storage media. Non-transitory computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the instructions set, storage media, processor or processing strategy and may be performed by software, hardware, integrated circuits, firmware, micro code, and the like, operating alone, or in combination. Likewise, processing strategies may include multiprocessing, multitasking, parallel processing, and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system. The memory 125 may store a model or machine learnt network.

The processor 123 is a general processor, central processing unit, control processor, graphics processing unit, digital signal processor, three-dimensional rendering processor, image processor, application specific integrated circuit, field programmable gate array, digital circuit, analog circuit, combinations thereof, or other now known or later developed device for processing medical imaging data. The processor 123 is a single device or multiple devices operating in serial, parallel, or separately. The processor 123 may be a main processor of a computer, such as a laptop or desktop computer, or may be a processor for handling some tasks in a larger system, such as in a server. The processor 123 is configured by instructions, design, hardware, and/or software to perform the acts discussed herein.

The processor 123 is configured to receive model parameters from a plurality of collaborator sites / clients / servers 131. The processor 123 is configured to aggregate the model parameters into a global model that is stored in the memory 125. The aggregation may be computed using weights that are calculated based on one or more metrics from each of the collaborators 131. The metrics may include, for example, a model divergence value, a class imbalance ratio, and/or a number of samples. The model divergence is estimated by measuring aggregation testing metrics for each collaborator 131 at each communication round. A preserved test dataset and a collaborator model from the current round are used to compute the global testing performance for each collaborator 131. In an example, model parameters may be weighted more from a collaborator 131 that has a low model divergence, a large amount of data samples, and an even class imbalance ratio. Similarly, model parameters may be weighted less during aggregation if the collaborator 131 uses few samples or where the respective model parameters diverge from the global model that was transmitted to the collaborators 131 after the last round. In this way, the aggregation mechanisms can take into account collaborators 131 that include high- or low-quality data, large amounts or small amounts of data, etc.

The processor 123 is configured to generate a global model that may eventually be applied for a certain task. The global model uses the aggregated weights and may be trained over multiple rounds of communications between the aggregation server 121 and the collaborators 131. The model may be locally trained using supervised or unsupervised learning. The model(s) may include a neural network that is defined as a plurality of sequential feature units or layers. Sequential is used to indicate the general flow of output feature values from one layer to input to a next layer. Sequential is used to indicate the general flow of output feature values from one layer to input to a next layer. The information from the next layer is fed to a next layer, and so on until the final output. The layers may only feed forward or may be bi-directional, including some feedback to a previous layer. The nodes of each layer or unit may connect with all or only a sub-set of nodes of a previous and/or subsequent layer or unit. Skip connections may be used, such as a layer outputting to the sequentially next layer as well as other layers. Rather than pre-programming the features and trying to relate the features to attributes, the deep architecture is defined to learn the features at different levels of abstraction based on the input data. The features are learned to reconstruct lower-level features (i.e., features at a more abstract or compressed level). Each node of the unit represents a feature. Different units are provided for learning different features. Various units or layers may be used, such as convolutional, pooling (e.g., max pooling), deconvolutional, fully connected, or other types of layers. Within a unit or layer, any number of nodes is provided. For example, 100 nodes are provided. Later or subsequent units may have more, fewer, or the same number of nodes. Unsupervised learning may also be used based on the distribution of the samples, using methods such as k-nearest neighbor.

Different neural network configurations and workflows may be used for or in the model such as a convolution neural network (CNN), deep belief nets (DBN), or other deep networks. CNN learns feed-forward mapping functions while DBN learns a generative model of data. In addition, CNN uses shared weights for all local regions while DBN is a fully connected network (e.g., including different weights for all regions of a feature map. The training of CNN is entirely discriminative through backpropagation. DBN, on the other hand, employs the layer-wise unsupervised training (e.g., pre-training) followed by the discriminative refinement with backpropagation if necessary. In an embodiment, the arrangement of the trained network is a fully convolutional network (FCN). Other network arrangements may be used, for example, a 3D Very Deep Convolutional Networks (3D-VGGNet). VGGNet stacks many layer blocks containing narrow convolutional layers followed by max pooling layers. A 3D Deep Residual Networks (3D-ResNet) architecture may be used. A Resnet uses residual blocks and skip connections to learn residual mapping.

The training data for the model includes ground truth data or gold standard data acquired at each collaborator 131 or site. Ground truth data and gold standard data is data that includes correct or reasonably accurate labels that are verified manually or by some other accurate method. The training data may be acquired at any point prior to inputting the training data into the model. Each local model may input the training data (e.g., patient data) and output a prediction or classification, for example. The prediction is compared to the annotations from the training data. A loss function may be used to identify the errors from the comparison. The loss function serves as a measurement of how far the current set of predictions are from the corresponding true values. Some examples of loss functions that may be used include Mean-Squared-Error, Root-Mean-Squared-Error, and Cross-entropy loss. Mean Squared Error loss, or MSE for short, is calculated as the average of the squared differences between the predicted and actual values. Root-Mean Squared Error is similarly calculated as the average of the root squared differences between the predicted and actual values. For cross-entropy loss each predicted probability is compared to the actual class output value (0 or 1) and a score is calculated that penalizes the probability based on the distance from the expected value. The penalty may be logarithmic, offering a small score for small differences (0.1 or 0.2) and enormous score for a large difference (0.9 or 1.0). During training and over repeated iterations, the network attempts to minimize the loss function as the result of a lower error between the actual and the predicted values means the network has done a good job in learning. Different optimization algorithms may be used to minimize the loss function, such as, for example, gradient descent, Stochastic gradient descent, Batch gradient descent, Mini-Batch gradient descent, among others. The process of inputting, outputting, comparing, and adjusting is repeated for a predetermined number of iterations with the goal of minimizing the loss function. Once adjusted and trained, the model is configured to be applied. In an embodiment, the trained model may be deployed to each of the collaborators 131. The collaborators 131 may apply the model for its intended use, for example, in a medical diagnosis or analysis.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention.

## Claims

1. A method for aggregating parameters from a plurality of collaborator devices (131) in a federated learning system that trains a model over multiple rounds of training, for each round of training the method comprising:

   receiving (A110) model parameters from two or more collaborator devices (131) of the plurality of collaborator devices (131);
   calculating (A120) for each of the two or more collaborator devices (131) a model divergence value that approximates how much an updated collaborator model for a respective collaborator device (131) of the two or more collaborator devices (131) deviates from a prior aggregated model;
   aggregating (A130) model parameters for the model from the received model parameters based at least on the respective model divergence value for each collaborator device (131); and
   transmitting (A140) the aggregated model parameters to the plurality of collaborator devices (131);
   wherein the aggregated model parameters are used by the plurality of collaborator devices (131) for a subsequent round of training.

2. The method of claim 1, further comprising:
   storing the aggregated model parameters as a preserved test dataset for calculating the model divergence value for the subsequent round of training.

3. The method of claim 1 or 2, wherein the model divergence value is calculated by an L2-norm of a difference between a respective updated collaborator model and the preserved test dataset.

4. The method of any one of claims claim 1 - 3, further comprising:
calculating a class imbalance ratio for each of the plurality of collaborator devices (131), wherein the model parameters are aggregated based further on the class imbalance ratios.

5. The method of any one of claims 1 - 4, further comprising:
determining a number of data samples of each of the plurality of collaborator devices (131), wherein the model parameters are aggregated based further on the number of data samples.

6. The method of any one of claims 1 - 5, wherein the model comprises a segmentation network configured to automatically quantify abnormal computed tomography patterns.

7. The method of any one of claims 1 - 6, wherein the plurality of collaborator devices (131) train the model using non-independently and identically distributed datasets.

8. The method of any one of claims 1 - 7, wherein the multiple rounds of training comprise more then ten rounds of training.

9. The method of any one of claims 1 - 8, wherein the model parameters comprise parameter vectors.

10. The method of any one of claims 1 - 9, wherein the plurality of collaborators (131) each comprise a hospital or medical center.

11. A system for federated learning, the system comprising:

a plurality of collaborators (131), each collaborator (131) of the plurality of collaborators (131) configured to train a local machine learned model using locally acquired training data, update local model weights for the local machine learned model, and send the updated local model weights to an aggregation server (121); and
the aggregation server (121) configured to receive the updated model weights from the plurality of collaborators, calculate a model divergence value for each collaborator (131) from respective updated model weights and a prior model, calculate aggregated model weights based at least in part on the model divergence values, and transmit the aggregated model weights to the plurality of collaborators (131) to update the local machine learned model.

12. The system of claim 11, wherein the aggregation server (121) is configured to carry out the method steps according to any one of claims 1 - 10, wherein the model weights are considered the model parameters.

13. The system of claim 11 or 12, wherein the plurality of collaborators (131) train the local machine learned model using non-independently and identically distributed locally acquired training data.

14. An aggregation server (121) for federated learning of a model, the aggregation server (121) comprising:

a transceiver (127) configured to communicate with a plurality of collaborator devices (131);
a memory (125) configured to store model parameters for the model; and
a processor (123) configured to receive model parameters from the plurality of collaborator devices (131), calculate for each collaborator device of the plurality of collaborator devices (131) a model divergence value, aggregate the model parameters from the plurality of collaborator devices at least in part based on the model divergence values, and transmit the aggregated model parameters to the plurality of collaborator devices (131).

15. The aggregation server (121) of claim 14, wherein the processor (123) is configured to carry pout the method of any one of claims 1 - 10.

Local Training

Collaborator 131

Collaborator 131

Collaborator 131

Local Model
Parameters

Aggregation Server
121

Global Model
Parameters

FIG. 1

A110 — Receive model parameters

A120 — Calculate divergence value

A130 — Aggregate model parameters

A140 — Transmit aggregated model parameters

FIG. 2

FIG. 3

Collaborators 131

Network
141

Aggregation Server 121

Transceiver
127

Processor
123

Memory
125

FIG. 4

Europäisches
Patentamt

European
Patent Office

Office européen
des brevets

**EUROPEAN SEARCH REPORT**

Application Number

**EP 22 19 7895**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO JIANHANG ET AL: "A Novel Server-side Aggregation Strategy for Federated Learning in Non-IID situations", 2021 20TH INTERNATIONAL SYMPOSIUM ON PARALLEL AND DISTRIBUTED COMPUTING (ISPDC), IEEE, 28 July 2021 (2021-07-28), pages 17-24, XP033966048, DOI: 10.1109/ISPDC52870.2021.9521631 [retrieved on 2021-08-23] * page 17, left-hand column, paragraph 2 - page 18, left-hand column, paragraph 1 * * page 19, left-hand column, paragraph 1 - right-hand column, paragraph 4; figure 2 * * page 18, left-hand column, paragraph 1 * * Algorithm 1; page 20, left-hand column, paragraph 1 * ----- | 1-15 | INV. G06N3/098 G06N3/084 G06N3/0464 G06N3/09 ADD. G16H30/40 |
| A | YEGANEH YOUSEF ET AL: "Inverse Distance Aggregation for Federated Learning with Non-IID Data", 26 September 2020 (2020-09-26), 16TH EUROPEAN CONFERENCE - COMPUTER VISION - ECCV 2020, CORNELL UNIVERSITY LIBRARY, 201 OLIN LIBRARY CORNELL UNIVERSITY ITHACA, NY 14853, PAGE(S) 150 - 159, XP047562901, [retrieved on 2020-09-26] * page 151, paragraph 1 * * page 152 - page 153; figures 1, 2 * * page 154, paragraph 1 * ----- -/-- | 1-15 | |

TECHNICAL FIELDS SEARCHED (IPC)

G06N
G16H

The present search report has been drawn up for all claims

| Place of search | Date of completion of the search | Examiner |
|---|---|---|
| Munich | 27 February 2023 | Herri, Edmond |

EPO FORM 1503 03.82 (P04C01)

Europäisches
Patentamt

European
Patent Office

Office européen
des brevets

**EUROPEAN SEARCH REPORT**

Application Number

EP 22 19 7895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZENG HUI ZENGHUI116@NUDT EDU CN ET AL: "FedCav: Contribution-aware Model Aggregation on Distributed Heterogeneous Data in Federated Learning", THE 2021 7TH INTERNATIONAL CONFERENCE ON INDUSTRIAL AND BUSINESS ENGINEERING, ACMPUB27, NEW YORK, NY, USA, 9 August 2021 (2021-08-09), pages 1-10, XP058864642, DOI: 10.1145/3472456.3472504 ISBN: 978-1-4503-9799-5 * page 4, left-hand column, paragraph 1 – page 6, right-hand column, paragraph 1 * ----- | 1-15 | |

**TECHNICAL FIELDS SEARCHED (IPC)**

The present search report has been drawn up for all claims

| Place of search | Date of completion of the search | Examiner |
|---|---|---|
| Munich | 27 February 2023 | Herri, Edmond |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................................
& : member of the same patent family, corresponding
document

EPO FORM 1503 03.82 (P04C01)